# FASCICULE DE BREVET EUROPEEN

(11) **EP 0 407 230 B1**
(45) Date de publication et mention de la délivrance du brevet: **05.04.1995**
(21) Numéro de dépôt: 90400985.9
(22) Date de dépôt: 10.04.1990
(51) Int. Cl.: C12N 15/30, A61K 39/015, G01N 33/569, C12Q 1/68, C12P 21/00

(54) **Protéine antigénique du stade sporozoite et hépatique de P. falciparum**
Antigen-Protein der Sporozoit- und hepatischen Phase von P. falciparum
Sporozoite and liver-stage protein antigen of P. falciparum

(30) Priorité: 12.04.1989 FR 8904847
(43) Date de publication de la demande: 09.01.1991
(73) Titulaire: INSTITUT PASTEUR, 75724 Paris Cédex 15 (FR)
(72) Inventeur: Druilhe, Pierre, F-94160 Saint-Mande (FR); Guerin-Marchand, Claudine, F-75012 Paris (FR)
(74) Mandataire: Desaix, Anne

(56) Documents cités:
- NATURE, vol. 329, no. 6135, 10-16 septembre 1987, pages 164-167, Basingstoke, Hampshire, GB; C. GUERIN-MARCHAND et al.: "A liver-stage-specific antigen of Plasmodium falciparum characterized by gene cloning"
- BIOFUTUR (+SUPPL.), no. 71, septembre 1988, pages 23-30, Paris, FR; G. LANGSLEY: "Paludisme: Vers un vaccin multivalent"
- BIOLOGICAL ABSTRACTS, vol. 78, 1984, résumé no. 59971, Biological Abstracts, Inc., Philadelphia, PA, US; P. DRUILHE et al.: "Species-specific and stage-specific antigens in exoerythrocytic stages of Plasmodium falciparum", & AM.J. TROP. MED. HYG. 33(3): 336-341. 1984
- BIOLOGICAL ABSTRACTS, vol. 86, 1988, résumé no. 49103, Biological Abstracts, Inc., Philadelphia, PA, US; A. SZARFMAN et al.: "Mature liver stages of cloned Plasmodium falciparum share epitopes with proteins from sporozoites andasexual blood stages", & PARASITE IMMUNOL. (OXF) 10(3): 339-351. 1988

## Description

Les parasites responsables du paludisme chez l'homme, dont notamment Plasmodium falciparum ou Plasmodium vivax pour ne citer que les principaux d'entre eux, présentent chez l'hôte humain des morphologies différentes et expriment des antigènes différents en fonction de leur localisation dans l'organisme de l'hôte infecté. Les différences morphologiques et antigéniques de ces parasites au cours de leurs cycles de vie chez l'homme, permettent de définir au moins quatre stades de développement distincts.

Le tout premier stade de développement du parasite chez l'homme correspond à la forme sporozoïte introduite dans le sang de l'hôte, par piqûres d'insectes porteurs du parasite. Le second stade correspond au passage du parasite dans le foie et à l'infection des cellules hépatiques dans lesquelles les parasites se développent pour former les schizontes hépatiques qui lorsqu'ils sont matures (6è jour après pénétration des sporozoïtes) libèrent par éclatement les mérozoïtes hépatiques. Le troisième stade est caractérisé par l'infection des érythrocytes sanguins par les formes asexuées (mérozoïtes) du parasite ; ce stade érythrocytaire de développement du parasite correspond à la phase pathogène de la maladie. Le quatrième stade correspond à la formation des formes à potentiel sexué (ou gamétocytes) qui deviendront des formes sexuées ou gamètes extracellulaires chez le moustique.

On sait que de nombreuses études ont été entreprises pour isoler à partir des souches de parasites infectantes pour un hôte humain des fractions polypeptidiques, d'une part pour assurer le diagnostic in vitro du paludisme par détection des anticorps correspondants, et, d'autre part, pour tenter de vacciner contre le paludisme.

Par exemple, des banques de cADNs clonés dérivés des sporozoïtes de Plasmodium falciparum ont été établies par ENEA et coll (1984) Science, vol. 225, 628-630. Il a été reconnu que ces banques comportaient des clones susceptibles d'exprimer des polypeptides immunogènes contenant des unités répétitives de 4 acides aminés spécifiques de l'antigène circumsporozoïtaire (de P. falciparum).

Toutefois, peu de travaux ont été effectués sur les formes hépatiques des parasites responsables du paludisme. La morphologie des formes hépatiques a été décrite pour la première fois en 1948 à partir de biopsies de volontaires humains infectés (Trans. Roy. Soc. Trop. Med. Hyg., 41, 785 (1948)). Un antigène spécifique du stade hépatique de P. falciparum a pu être décrit dans le foie de singes d'Amérique du Sud insensibles aux formes sanguines du parasite, mais chez lesquels les formes hépatiques peuvent se développer (Am. J. Trop. Med. Hyg., 33, (3) 336-341 (1984)).

La détection de la localisation des antigènes spécifiques du foie a été réalisée par immunofluorescence tout au long des étapes de maturation du schizonte. Ils sont localisés à la périphérie du parasite de taille 5 à 40 microns ; par la suite ils sont distribués entre les cytomères ou paquets de mérozoïtes, lorsque les schizontes atteignent entre 50 et 100 microns. Ils se distinguent des antigènes de surface des sporozoïtes et des antigènes partagés par les schizontes du sang et du foie qui donnent une image d'immunofluorescence interne au parasite.

Bien qu'il soit désormais possible de cultiver des formes hépatiques de P.falciparum dans des hépatocytes humains (Science, 227, 440 (1985)), le faible taux de sporozoïtes se transformant en formes matures du parasite par les méthodes de culture in vitro et in vivo ne permet pas l'analyse biochimique des antigènes produits au stade hépatique.

Il a également été observé que les individus atteints de paludisme possédent un taux très élevé d'anticorps dirigés contre les antigènes hépatiques. Ces antigènes hépatiques semblent être des immunogènes très puissants, parmi les plus puissants de tous les antigènes synthétisés aux différents stades de développement du parasite. Par contre, le taux d'anticorps dirigés contre les formes des stades sanguins du parasite est parfois très faible, et de ce fait le diagnostic réalisé à partir d'antigènes spécifiques des stades sanguins peut parfois être faussement négatif.

Un des buts de la présente invention est précisément de permettre le diagnostic in vitro de l'infection d'un individu par P. falciparum dans des conditions plus sensibles que ne le permettent les méthodes actuelles.

L'invention a également pour objet de nouvelles compositions pour la vaccination chez l'homme contre le paludisme provoqué par P. falciparum.

L'invention concerne plus particulièrement des molécules, polypeptides ou compositions peptidiques ou polypeptidiques, caractérisées par la présence dans leur structure d'une ou de plusieurs séquences peptidiques porteuses d'un ou plusieurs épitopes caractéristiques des protéines soit présentes en surface des sporozoïtes, soit résultant de l'activité infectieuse de P. falciparum dans les cellules hépatiques.

Une molécule exprimée spécifiquement au cours de la phase hépatique a été identifiée par criblage avec des serums polyclonaux d'une banque d'ADN génomique clonée dans un vecteur d'expression (GUERIN-MARCHAND, C. et al ; Nature, 329, 164-167,(1987)) Cette molécule LSA (Liver Stage Antigen) est constituée de motifs répétitifs de 17 acides aminés et semble très immunogène dans les conditions naturelles d'exposition à la maladie.

La molécule polypeptidique objet de la présente invention en diffère par l'absence de répétitions dans la séquence reconnue par des anticorps, et par son expression aussi bien au niveau du stade sporozoïte que du stade hépatique.

Il sera fait référence dans ce qui suit aux dessins dans lesquels
- la figure 1 fournit la séquence nucléotidique codant pour le polypeptide de 87 acides aminés caractéristique des formes sporozoïtes et des formes hépatiques de P. falciparum ;
- la figure 2 fournit la séquence nucléotidique de la figure 1 ainsi que la séquence complémentaire de cette dernière, et une carte de restriction de cette séquence d'ADN,
- la figure 3 représente les séquences nucléotidiques I et II issues de la séquence de nucléotides de la figure 1 et utilisables en tant qu'amorces d'ADN ou en tant que sondes,
- la figure 4 représente la révélation en immunotransfert, à partir d'un extrait de sporozoïtes (souche NF54), de la protéine parasitaire comportant la séquence polypeptidique de l'invention (4è colonne ; polypeptide de 70 kD désigné par DG671 ou SALSA), par rapport à divers témoins : sérum total (SHI2 et SHI5), anticorps monoclonal dirigé contre le tétrapeptide répétitif de la circumsporozoïte protéine (Mab), anticorps anti-LSA (DG307°,
- la figure 5 représente la localisation de la protéine SALSA en surface du sporozoïte : marquage, en microcospie électronique de coupes de sporozoïtes (souche NF54), par des anticorps anti-SALSA (DG671) révélé par un deuxième anticorps marqué à l'or colloïdal. Témoins : sérum total (SHI2) et anticorps anti-LSA (control).

Un polypeptide selon l'invention est essentiellement caractérisé en ce qu'il comporte au moins une séquence peptidique porteuse d'un, ou plusieurs, épitope caractéristique d'une protéine produite au stade sporozoïte et dans les hépatocytes infectés par P. falciparum, cette séquence comprenant une succession d'environ 10 acides aminés au nombre maximum d'acides aminés de l'enchaînement peptidique suivant : 
Glu-Phe-Arg-Val-Ser-Thr-Ser-Asp-Thr-Pro-Gly-Gly-Asn-
1 13
Glu-Ser-Ser-Ser-Ala-Phe-Pro-Gln-Phe-Ile-Trp-Ser-Ala-
14 26
Glu-Lys-Lys-Asp-Glu-Lys-Glu-Ala-Ser-Glu-Gln-Gly-Glu-
27 39
Glu-Ser-His-Lys-Lys-Glu-Asn-Ser-Gln-Glu-Ser-Ala-Asn-
40 52
Gly-Lys-Asp-Asp-Val-Lys-Glu-Glu-Lys-Lys-Thr-Asn-Glu-
53 65
Lys-Lys-Asp-Asp-Gly-Lys-Thr-Asp-Lys-Val-Gln-Glu-Lys-
66 78
Val-Leu-Glu-Lys-Ser-Pro-Lys-Glu-Phe.
79 87
et dans laquelle "Leu" est la leucine, "Ser" est la sérine, "Lys" est la lysine, "Glu" est l'acide glutamique, "Gln" est la glutamine, "Asp" est l'acide aspartique, "Phe" est la phénylalanine, "Val" est la valine, "Thr" est la thréonine, "Pro" est la proline, "Gly" est la glycine, "Asn" est l'asparagine, "Ala" est l'alanine, "Ile" est l'isoleucine, "Trp" est le tryptophane, "His" l'histidine, "Leu" est la leucine, et "Arg" est l'arginine.

L'invention concerne en premier lieu des peptides monomères comprenant la séquence peptidique unique de 87 acides aminés répondant à la formule sus-indiquée, et dont les acides aminés terminaux possédent des extrémités respectivement amine et carboxylique libres, ou des oligomères contenant notamment des multiples de la séquence peptidique de 87 acides aminés sus- mentionnée.

L'invention concerne également le polypeptide de 87 acides aminés lui-même, caractérisé par l'enchaînement d'acides aminés sus-mentionné.

Cette séquence peptidique de 87 acides aminés possède la caractéristique d'être reconnue par des anticorps reconnaissant les stades sporozoïte et hépatique de P. falciparum ; cette séquence de 87 acides aminés n'est pas reconnue par des anticorps reconnaissant les stades sanguins (érythrocytaires) de P.falciparum.

L'invention a également pour objet toute séquence peptidique issue de la séquence de 87 acides aminés sus-mentionnée, (parfois désignée ci-après par sous-séquence peptidique) et constituée d'une succession de 5, et de préférence de 10 à 86 acides aminés comprise dans l'enchaînement peptidique de 87 acides aminés sus-mentionné. Ces sous-séquences peptidiques possèdent des caractéristiques antigéniques analogues à celles décrites ci-dessus pour le polypeptide de 87 acides aminés.

Un procédé de sélection d'une sous-séquence peptidique de l'invention consiste à vérifier que cette sous-séquence est reconnue par des immunsérums humains provenant d'individus ayant été infectés par P.falciparum.

A titre d'exemple de sous-séquences peptidiques de l'invention, on peut citer notamment les polypeptides délimités respectivement par les acides aminés situés aux positions 1 à 5, 20 à 50, 52 à 65, et 72 à 80 de l'enchaînement peptidique sus-mentionné.

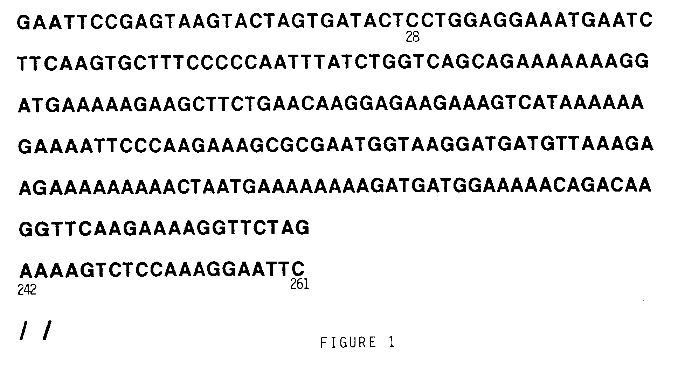

Deux sous-séquences peptidiques particulièrement intéressantes, ci-après désignées par SALSA1 et SALSA2, sont représentées par les enchaînements d'acides aminés suivantes :
1-SALSA1 : 
Ser-Ala-Glu-Lys-Lys-Asp-Glu-Lys-Glu-Ala-Ser-Glu-Gln-
Gly-Glu-Glu-Ser-His-Lys-Lys-Glu-Asn-Ser-Gln-Glu-Ser-
Ala,
ou encore représentée de la manière suivante
SAEKKDEKEASEQGEESHKKENSQESA
2 - SALSA2 : 
Asn-Gly-Lys-Asp-Asp-Val-Lys-Glu-Glu-Lys-Lys-Thr-Asn-
Glu-Lys-Lys-Asp-Asp-Gly-Lys-Thr-Asp-Lys-Val-Gln-Glu-
Lys-Val-Leu-Glu-Lys-Ser-Pro-Lys-Glu-Phe,
ou encore représentée de la manière suivante :
NGKDDVKEEKKTNEKKDDGKTDKVQEKVLEKSPKEF
D'une manière générale, l'invention concerne tout polypeptide comprenant de 10 à 87 acides aminés décrits ci-dessus, et codé par l'acide nucléique représenté sur la figure 1.

Il va de soi que les fonctions réactives libres que sont susceptibles de posséder certains acides aminés entrant dans la constitution des polypeptides selon l'invention, notamment les groupes carboxyles libres portés par les groupes Glu ou par l'acide aminé C-terminal, d'une part, et/ou les groupes libres portés par l'acide aminé N-terminal ou par des acides aminés intérieurs à la chaîne peptidique, par exemple Lys, d'autre part peuvent être modifiées, dès lors que cette modification n'entraîne pas une modification des propriétés antigéniques, le cas échéant immunogène, de l'ensemble du polypeptide. Les molécules ainsi modifiées entrent naturellement dans le cadre de la protection donnée à l'invention par les revendications. Ces fonctions carboxyles sont éventuellement acylées ou estérifiées.

D'autres modifications entrent également dans le cadre de l'invention. En particulier, les fonctions amine ou ester, ou les deux à la fois, des acides aminés terminaux peuvent être engagées elles-mêmes dans des liaisons avec d'autres acides aminés. Par exemple l'acide N-terminal peut être lié à une séquence comprenant de un à plusieurs acides aminés correspondant à une partie de la région C-terminale d'un autre peptide conforme à la définition qui en a été donnée plus haut, ou vice-versa.

Il va de soi également que toute séquence peptidique issue de la modification, par substitution et/ou par addition et/ou suppression d'un ou plusieurs acides aminés, de la séquence peptidique de 87 acides aminés, ou d'une sous-séquence peptidique selon l'invention, entre dans le cadre de la protection donnée à l'invention par les revendications, dès lors que cette modification n'altère pas les propriétés antigéniques ou immunogènes dudit polypeptide, notamment lorsque ces propriétés immunogènes ont été renforcées de façon adéquate, par exemple par association du polypeptide avec un adjuvant immunologique approprié (par exemple un muramylpeptide) ou par couplage avec une molécule porteuse de poids moléculaire plus élevé (par exemple une sérum-albumine ou une poly-lysine) ou une toxine du type tétanique ou un autre antigène de P.falciparum.

L'invention concerne plus généralement tout polypeptide caractérisé par la présence dans sa structure d'une ou plusieurs séquences peptidiques présentant des réactions immunologiques croisées avec tout ou partie de la séquence peptidique répondant à la formule précédente, ou sous-séquence peptidique selon l'invention, vis-à-vis des anticorps inductibles par cette dernière in vivo.

L'invention concerne également toute séquence de nucléotides codant pour un polypeptide de l'invention.

L'invention a plus particulièrement pour objet la séquence nucléotidique, constitué des 261 nucléotides, représentés sur la figure 1, et codant pour le polypeptide de 87 acides aminés sus-mentionné.

L'invention concerne aussi les séquences nucléotidiques codant pour des sous-séquences peptidiques de l'invention. A titre d'exemple, on citera les séquences nucléotidiques délimitées respectivement par les nucléotides situés aux positions 1 à 45, 60 à 150, 156 à 195, et 216 à 240 de la figure 1.

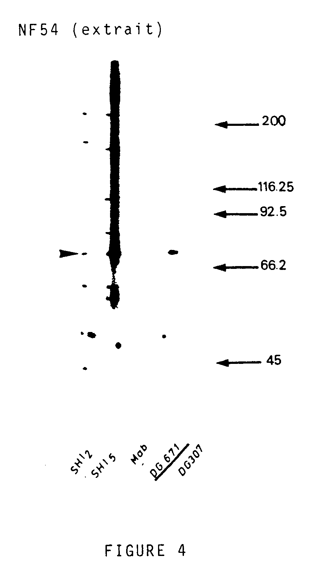

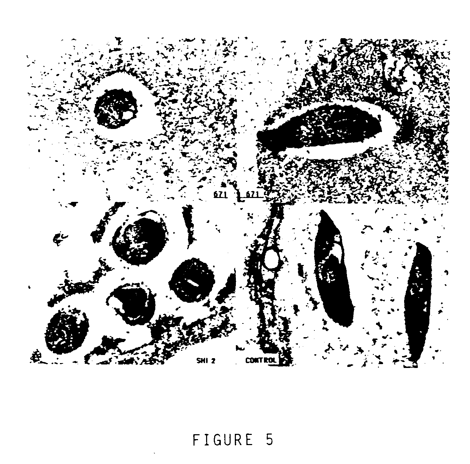

L'invention concerne également toute séquence de nucléotides codant pour un polypeptide identique, ou analogue, tant du point de vue de la structure que des caractéristiques antigéniques, à ceux de l'invention, cette séquence étant capable de s'hybrider avec la séquence nucléotidique de la figure 1, ou la séquence complémentaire de cette dernière, dans les conditions suivantes :
- pré-traitement (pré-hybridation) du filtre de nitrocellulose supportant le fragment d'acide nucléique à tester avec le tampon d'hybridation, (composé de 6 x SSC, 2,5 % de lait, 0,5 % de SDS), cette opération étant effectuée à 65° C pendant 1 heure ;
- remplacement du tampon d'hybridation au contact du support, sur lequel le fragment d'acide nucléique est alors fixé, par du tampon d'hybridation de même composition (comprenant en outre de l'ADN de saumon dénaturé), et addition de la séquence de la figure 1 en tant que sonde, notamment marquée radioactivement, et préalablement dénaturée par un traitement à 100°C pendant 5 minutes ;
- incubation dudit fragment d'acide nucléique fixé sur le support dans ce tampon d'incubation avec la séquence de la figure 1 à 65° C pendant une durée de 16 à 24 heures,
- l'élimination du tampon contenant la sonde non fixée, par 3 lavages successifs de 5 minutes chacun avec 2 x SSC, 0,1 % SDS à 65°C, suivis de 3 lavages successifs de 5 minutes chacun avec 0,1 x SSC, 0,1 % SDS à 65°C.

Il est à rappeler que 1 x SSC est constitué de 0,15 M de NaCl et 0,01 M de citrate de sodium, pH 7 ; le SDS est le sodium dodécyl sulfate.

L'invention a également pour objet tout acide nucléique recombinant contenant au moins une séquence de nucléotides de l'invention, insérée dans un acide nucléique hétérologue vis-à-vis de ladite séquence de nucléotides.

L'invention concerne plus particulièrement un acide nucléique recombinant tel que défini ci-dessus, dans lequel la séquence de nucléotides de l'invention est précédée d'un promoteur (notamment un promoteur inductible) sous le contrôle duquel la transcription de ladite séquence est susceptible d'être effectuée et, le cas échéant, suivie d'une séquence codant pour des signaux de terminaison de la transcription.

L'invention concerne tout vecteur recombinant, utilisé en particulier pour le clonage d'une séquence nucléotidique de l'invention, et/ou l'expression du polypeptide codé par cette séquence, et caractérisé en ce qu'il contient un acide nucléique recombinant, tel que défini ci-dessus, en l'un de ses sites non essentiel pour sa réplication.

A titre d'exemple de vecteur sus-mentionné, on citera les plasmides, les cosmides, ou les phages.

A ce titre, l'invention concerne plus particulièrement le plasmide DG 671 déposé à la C.N.C.M sous le numéro I-855 le 31 mars 1989.

L'invention a également pour objet un procédé de préparation d'un polypeptide de l'invention, par transformation d'un hôte cellulaire à l'aide d'un vecteur recombinant de type sus-indiqué, suivie de la mise en culture de l'hôte cellulaire ainsi transformé, et de la récupération du polypeptide dans le milieu de culture.

Ainsi, l'invention concerne tout hôte cellulaire transformé par un vecteur recombinant tel que défini ci-dessus, et comprenant les éléments de régulation permettant l'expression de la séquence de nucléotides codant pour un polypeptide selon l'invention.

L'invention a plus particulièrement pour objet des amorces d'ADN (ou d'ARN) utilisables dans le cadre de la synthèse de séquences nucléotidiques et/ou polypeptides selon l'invention, par la technique du PCR (Polymerase Chain Reaction) telle que décrite dans les brevets américains n° 4,683,202 et n°4,683,195 et de la demande de brevet européenne n° 200.362 (PCR = amplification en chaîne de l'ADN).

L'invention concerne toute amorce d'ADN ou d'ARN, caractérisée en ce qu'elle est constituée d'environ 10 à 25 nucléotides, identiques aux 10 à 25 premiers nucléotides de la séquence de nucléotides codant pour une séquence peptidique selon l'invention ou identiques aux 10 à 25 derniers nucléotides de ladite séquence.

L'invention concerne également toute amorce d'ADN ou d'ARN, caractérisée en ce qu'elle est constituée d'environ 10 à 25 nucléotides complémentaires des 10 à 25 premiers nucléotides de la séquence nucléotidique codant pour une séquence peptidique selon l'invention ou complémentaire des 10 à 25 derniers nucléotides de ladite séquence de nucléotides.

L'invention a également pour objet toute amorce d'ADN ou d'ARN, caractérisée en ce qu'elle est constituée d'environ 10 à 25 nucléotides capables de s'hybrider avec les 10 à 25 premiers nucléotides ou avec les 10 à 25 derniers nucléotides de ladite séquence de nucléotides codant pour un polypeptide de l'invention, dans les conditions d'hybridation définies ci-dessus.

Ainsi la présente invention concerne plus particulièrement un procédé de préparation d'un polypeptide de l'invention comprenant les étapes suivantes ;
- le cas échéant, l'amplification préalable suivant la technique PCR de la quantité de séquences de nucléotides codant pour ledit polypeptide à l'aide de deux amorces d'ADN choisies de manière à ce que l'une de ces amorces soit identique aux 10 à 25 premiers nucléotides de la séquence nucléotidique codant pour ledit polypeptide, tandis que l'autre amorce est complémentaire des 10 à 25 derniers nucléotides (ou s'hybride avec ces 10 à 25 derniers nucléotides) de ladite séquence nucléotidique, ou inversement de manière à ce ce que l'une de ces amorces soit identique aux 10 à 25 derniers nucléotides de ladite séquence, tandis que l'autre amorce est complémentaire des 10 à 25 premiers nucléotides (ou s'hybride avec les 10 à 25 premiers nucléotides) de ladite séquence nucléotidique,
   suivie de l'introduction desdites séquences de nucléotides ainsi amplifiées dans un vecteur approprié,
- la mise en culture, dans un milieu de culture approprié, d'un hôte cellulaire préalablement transformé par un vecteur approprié contenant un acide nucléique selon l'invention comprenant la séquence nucléotidique codant pour ledit polypeptide, et
- la récupération, à partir du susdit milieu de culture du polypeptide produit par ledit hôte cellulaire transformé.

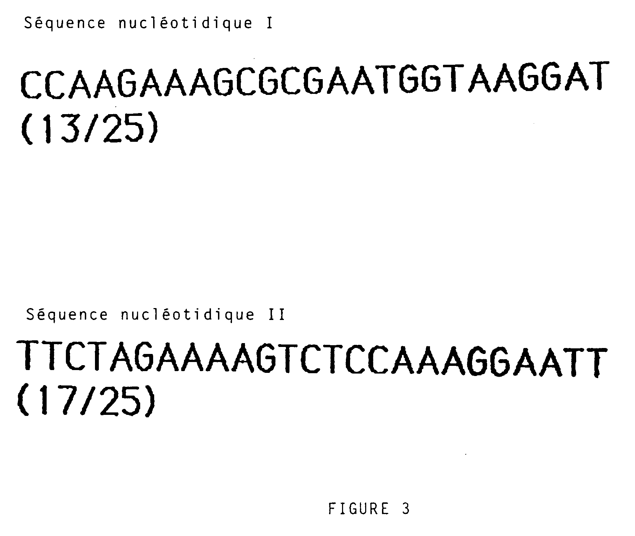

A titre d'exemple d'amorces d'ADN ou d'ARN selon l'invention, on citera les séquences I et II représentées sur la figure 3.

Les peptides selon l'invention peuvent être préparés par les techniques classiques, dans le domaine de la synthèse des peptides. Cette synthèse peut être réalisée en solution homogène ou en phase solide.

Par exemple, on aura recours à la technique de synthèse en solution homogène décrit par HOUBENWEYL dans l'ouvrage intitulé "Methode der Organischen Chemie" (Méthode de la Chimie Organique) édité par E. Wunsch, vol. 15-I et II., THIEME, Stuttgart 1974.

Cette méthode de synthèse consiste à condenser successivement deux-à-deux les aminoacyles successifs dans l'ordre requis, ou à condenser des aminoacyles et des fragments préalablement formés et contenant déjà plusieurs aminoacyles dans l'ordre approprié, ou encore plusieurs fragments préalablement ainsi préparés, étant entendu que l'on aura eu soin de protéger au préalable toutes les fonctions réactives portées par ces aminoacyles ou fragments, à l'exception des fonctions amines de l'un et carboxyles de l'autre ou vice-versa, qui doivent normalement intervenir dans la formation des liaisons peptidiques, notamment après activation de la fonction carboxyle, selon les méthodes bien connues dans la synthèse des peptides. En variante, on pourra avoir recours à des réactions de couplage mettant en jeu des réactifs de couplage classique, du type carbodiimide, tels que par exemple la 1-ethyl-3-(3-diméthyl-aminopropyl)-carbodiimide.

Lorsque l'aminoacyle mis en oeuvre possède une fonction acide supplémentaire (notamment dans le cas de l'acide glutamique), ces fonctions seront protégées, par exemple par des groupes t-butylester.

Dans le cas de la synthèse progressive, acide aminé par acide aminé, la synthèse débute de préférence par la condensation de l'amino-acide C-terminal avec l'aminoacide qui correspond à l'aminoacyle voisin dans la séquence désirée et ainsi de suite, de proche en proche, jusqu'à l'acide aminé N-terminal. Selon une autre technique préférée de l'invention, on a recours à celle décrite par R.D. MERRIFIELD dans l'article intitulé "Solid phase peptide synthesis" (J. Am. Soc., 45, 2149-2154).

Pour fabriquer une chaîne peptidique selon le procédé de MERRIFIELD, on a recours à une résine polymère très poreuse, sur laquelle on fixe le premier acide aminé C-terminal de la chaîne. Cet acide aminé est fixé sur la résine par l'intermédiaire de son groupe carboxylique et sa fonction amine est protégée, par exemple par le groupe t-butyloxycarbonyle.

Lorsque le premier acide aminé C-terminal est ainsi fixé sur la résine, on enlève le groupe protecteur de la fonction amine en lavant la résine avec un acide.

Dans le cas où le groupe protecteur de la fonction amine est le groupe t-butyloxycarbonyle, il peut être éliminé par traitement de la résine à l'aide d'acide trifluoroacétique.

On couple ensuite le deuxième acide aminé qui fournit le second amino-acyle de la séquence recherchée, à partir du résidu amino-acyle C-terminal sur la fonction amine déprotégée du premier acide aminé C-terminal fixé sur la chaîne. De préférence, la fonction carboxyle de ce deuxième acide aminé est activée, par exemple par la dicyclohexylcarbodiimide, et la fonction amine est protégée, par exemple par le t-butyloxycarbonyle.

On obtient ainsi la première partie de la chaîne peptidique recherchée, qui comporte deux acide aminés, et dont la fonction amine terminale est protégée. Comme précédemment, on déprotège la fonction amine et on peut ensuite procéder à la fixation du troisième aminoacyle, dans les conditions analogues à celles de l'addition du deuxième acide aminé C-terminal.

On fixe ainsi, les uns après les autres, les acides aminés qui vont constituer la chaîne peptidique sur le groupe amine chaque fois déprotégé au préalable de la portion de la chaîne peptidique déjà formée, et qui est rattachée à la résine.

Lorsque la totalité de la chaîne peptidique désirée est formée, on élimine les groupes protecteurs des différents acides aminés constituant la chaîne peptidique et on détache le peptide de la résine par exemple à l'aide d'acide fluorydrique.

L'invention concerne également les oligomères hydrosolubles des peptides monomères sus-indiqués.

L'oligomérisation peut provoquer un accroissement de l'immunogénicité des peptides monomères selon l'invention. Sans qu'une telle indication chiffrée puisse être considérée comme limitative, on mentionnera néanmoins que ces oligomères peuvent, par exemple, contenir de 2 à 10 unités monomères.

On peut avoir recours, pour réaliser l'oligomérisation, à toute technique de polymérisation couramment utilisée dans le domaine des peptides, cette polymérisation étant conduite jusqu'à l'obtention d'un oligomère ou polymère contenant le nombre de motifs monomères requis pour l'acquisition de l'immunogénicité désirée.

Une méthode d'oligomérisation ou de polymérisation du monomère consiste dans la réaction de celui-ci avec un agent de réticulation tel que le glutaraldéhyde.

On peut également avoir recours à d'autres méthodes d'oligomérisation ou de couplage, par exemple à celle mettant en jeu des couplages successifs d'unités monomères, par l'intermédiaire de leurs fonctions terminales carboxyle et amine en présence d'agents de couplage homo- ou hétéro- bifonctionnels.

L'invention concerne encore les conjugués obtenus par couplage covalent des peptides selon l'invention (ou des susdits oligomères) à des molécules porteuses (naturelles ou synthétiques), physiologiquement acceptables et non toxiques, par l'intermédiaire de groupements réactifs complémentaires respectivement portés par la molécule porteuse et le peptide. Des exemples de groupements appropriés sont illustrés dans ce qui suit.

A titre d'exemple de molécules porteuses ou supports macromoléculaires entrant dans la constitution des conjugués selon l'invention, on mentionnera des protéines naturelles, telles que l'anatoxine tétanique, l'ovalbulmine, des sérums albumines, des hémocyamines, le PPD de la tuberculine ("Purified Protein Derivative" = PPD) etc...

A titre de supports macromoléculaires synthétiques, on mentionnera par exemple des polylysines ou des poly(D-L-alanine)-poly(L-lysine).

La littérature mentionne d'autres types de supports macromoléculaires susceptibles d'être utilisés, lesquels présentent en général un poids moléculaire supérieur à 20 000.

Pour synthétiser les conjugués selon l'invention, on peut avoir recours à des procédés connus en soi, tels que celui décrit par FRANTZ et ROBERTSON dans Infect. and Immunity, 33, 193-198 (1981), ou celui décrit dans Applied and Environmental Microbiology, (octobre 1981), vol. 42, n° 4, 611-614 par P.E. KAUFFMAN en utilisant le peptide et la molécule porteuse appropriée.

Dans la pratique, on utilisera avantageusement comme agent de couplage les composés suivants, cités à titre non limitatif : aldéhyde glutarique, chloroformiate d'éthyle, carbodiimides hydrosolubles [N′(3-diméthylamino-propyl) carbodiimide, HCl], diisocyanates, bis-diazobenzidine, di- et trichloro-s-triazines, bromures de cyanogène, ainsi que les agents de couplage mentionnés dans Scand. J. Immunol., (1978), vol. 8, p. 7-23 (AVRAMEAS, TERNYNCK, GUESDON).

On peut avoir recours à tout procédé de couplage faisant intervenir d'une part une ou plusieurs fonctions réactives du peptide et d'autre part, une ou plusieurs fonctions réactives de molécules supports. Avantageusement, il s'agit des fonctions carboxyle et amine, lesquelles peuvent donner lieu à une réaction de couplage en présence d'un agent de couplage du genre de ceux utilisés dans la synthèse des protéines, par exemple,le 1-éthyl-3-(3-diméthylaminopropyl)-carbodi- imide, le N-hydroxybenzotriazole, etc... On peut encore avoir recours à la glutaraldéhyde, notamment lorsqu'il s'agit de relier entre eux des groupes aminés respectivement portés par le peptide et la molécule support.

Les acides nucléiques de l'invention peuvent être préparés soit par un procédé chimique, soit par d'autres procédés.

Un mode de préparation approprié des acides nucléiques comportant au maximum 200 nucléotides (ou 200 pb, lorsqu'il s'agit d'acides nucléiques bicaténaires) de l'invention comprend les étapes suivantes :
- la synthèse d'ADN en utilisant la méthode automatisée des β-cyanethylphosphoramidite décrite dans Bioorganic Chemistry 4; 274-325 (1986),
- le clonage des acides nucléiques ainsi obtenus dans un vecteur approprié et la récupération de l'acide nucléique par hybridation avec une sonde appropriée.

Un mode de préparation, par voie chimique, d'acides nucléiques de longueur supérieure à 200 nucléotides (ou 200pb, lorsqu'il s'agit d'acides nucléiques bicaténaires) de l'invention comprend les étapes suivantes :
- l'assemblage d'oligonucléotides synthétisés chimiquement, pourvus à leurs extrémités de sites de restriction différents, dont les séquences sont compatibles avec l'enchaînement en acides aminés du polypeptide naturel selon le principe décrit dans Proc. Natl. Acad. Sci. USA, 80; 7461-7465, (1983),
- le clonage des acides nucléiques ainsi obtenus dans un vecteur approprié et la récupération de l'acide nucléique recherché par hybridation avec une sonde appropriée.

Les acides nucléiques de l'invention peuvent également être préparés de la manière suivante :
- incubation de l'ADN génomique, isolé à partir d'une souche de P. falciparum, avec de l'ADNase I, puis addition d'EDTA et purification par extraction au mélange phenol/chloroforme/alcool isoamylique (25/24/1) puis par l'éther,
- traitement de l'ADN ainsi extrait par de l'Eco R1 méthylase en présence de DTT, et purification par extraction telle que décrite ci-dessus,
- incubation de l'ADN ainsi purifié avec les 4 désoxynucléotides triphosphates dATP, dCTP, dGTP, et dTTP en présence de T4 ADN polymérase et d'ADN ligase de E. coli, puis purification selon la méthode décrite ci-dessus,
- le clonage des acides nucléiques ainsi obtenus dans un vecteur approprié et la récupération de l'acide nucléique recherché à l'aide d'une sonde appropriée.

Les sondes nucléotidiques utilisées pour la récupération de l'acide nucléique recherché dans les procédés sus-mentionnés, sont constituées généralement de 40 à 200 nucléotides de la séquence nucléotidique représentée sur la figure 1, ou sa séquence complémentaire, et sont susceptibles de s'hybrider avec l'acide nucléique recherché dans les conditions d'hybridation définies ci-dessus. La synthèse de ces sondes est effectuée selon la méthode automatisée des β-cyanethylphosphoramidite décrite dans Bioorganic Chemistry **4**, 274-325 (1986).

Outre le fait qu'ils soient présents au niveau du sporozoïte, les polypeptides selon l'invention possèdent également des propriétés antigéniques caractéristiques des antigènes spécifiques du stade hépatique du développement de P.falciparum.

En effet, comme il le sera plus particulièrement décrit à l'aide d'exemples de molécules polypeptidiques selon l'invention dans la description détaillée qui suit, les polypeptides selon l'invention réagissent spécifiquement avec les anticorps dirigés contre les antigènes hépatiques produits par P. falciparum, et également avec les anticorps dirigés contre d'autres antigènes de sporozoïte de P. falciparum, mais pas avec les anticorps dirigés contre des antigènes produits à d'autres stades ou par d'autres espèces de Plasmodium.

Ces polypeptides selon l'invention reconnaissent donc spécifiquement les anticorps produits par le système immunitaire d'un individu infecté par P. falciparum sous l'effet des antigènes de stade hépatique dont le caractère fortement immunogène a été précédemment mentionné.

Ainsi la possibilité de production en grande quantité des molécules selon l'invention ainsi que leurs propriétés de reconnaissance spécifique des anticorps les plus activement produits lors de l'infection d'un individu par P. falciparum, font desdites molécules des réactifs de choix pour le diagnostic in vitro du paludisme chez un individu infecté par P. falciparum.

L'invention concerne donc un procédé de détection in vitro d'anticorps corrélables au paludisme issu de l'infection d'un individu par P. falciparum dans un tissu ou fluide biologique susceptible de les contenir, ce procédé comprenant la mise en contact de ce tissu ou fluide biologique avec une molécule selon l'invention dans des conditions permettant une réaction immunologique in vitro entre lesdites molécules et les anticorps éventuellement présents dans le tissu ou fluide biologique, et la détection in vitro du complexe antigène-anticorps éventuellement formé.

De préférence, le milieu biologique est constitué par un sérum humain.

Toute procédure classique peut être mise en oeuvre pour réaliser une telle détection.

A titre d'exemple une méthode préférée met en jeu des processus immunoenzymatiques selon la technique ELISA, ou immunofluorescents, ou radioimmunologiques (RIA) ou équivalent.

Ainsi l'invention concerne également tout polypeptide selon l'invention marqué à l'aide d'un marqueur adéquat du type enzymatique, fluorescent, radioactif, etc...

De telles méthodes comprennent par exemple les étapes suivantes :
- dépôt de quantités déterminées d'une composition polypeptidique selon l'invention dans les puits d'une microplaque de titration,
- introduction dans lesdits puits de dilutions croissantes du sérum devant être diagnostiqué,
- incubation de la microplaque,
- rinçages répétés de la microplaque,
- introduction dans les puits de la microplaque d'anticorps marqués contre des immunoglobulines du sang,
   le marquage de ces anticorps ayant été réalisé à l'aide d'une enzyme sélectionnée parmi celles qui sont capables d'hydrolyser un substrat en modifiant l'absorption des radiations de ce dernier, au moins à une longueur d'onde déterminée,
- détection, en comparaison avec un témoin de contrôle, de la quantité de substrat hydrolysé.

L'invention concerne également des coffrets ou nécessaires, ou kits pour le diagnostic in vitro du paludisme provoqué par P. falciparum qui comprennent :
- une composition polypeptidique selon l'invention,
- les réactifs pour la constitution du milieu propice à la réalisation de la réaction immunologique,
- les réactifs permettant la détection du complexe antigènes-anticorps produit par la réaction immunologique. De tels réactifs peuvent également porter un marqueur, ou être susceptibles d'être reconnus à leur tour par un réactif marqué. Plus particulièrement dans le cas où la composition polypeptidique sus-mentionnée n'est pas marquée.
- un tissu ou fluide biologique de référence dépourvu d'anticorps reconnus par la composition polypeptidique sus-mentionnée,
L'invention concerne les anticorps eux-mêmes formés contre les polypeptides de l'invention, et obtenus par immunisation d'un animal avec ces polypeptides suivie de la récupération des anticorps formés.

Il va de soi que cette production n'est pas limitée aux anticorps polyclonaux.

Elle s'applique encore à tout anticorps monoclonal produit par tout hybridome susceptible d'être formé, par des méthodes classiques, à partir des cellules spléniques d'un animal, notamment de souris ou de rat, immunisés contre l'un des polypeptides purifiés de l'invention, d'une part et des cellules d'une lignée de cellule myélome approprié d'autre part, et d'être sélectionné, par sa capacité à produire des anticorps monoclonaux reconnaissant le polypeptide initialement mis en oeuvre pour l'immunisation des animaux.

Les anticorps de l'invention peuvent se révéler particulièrement utiles pour la détection des sporozoïtes chez les moustiques.

L'invention concerne également une sonde nucléotidique de détection caractérisée en ce qu'elle est constituée par tout ou partie d'une des séquences de nucléotides telles que définies ci-dessus de l'invention.

L'invention a plus particulièrement pour objet une méthode de diagnostic in vitro du paludisme chez un individu susceptible d'être infecté par P.falciparum qui comprend les étapes suivants :
- éventuellement l'amplification préalable de la quantité de séquences de nucléotides selon l'invention, susceptibles d'être contenues dans l'échantillon biologique prélevé chez ledit individu, à l'aide de deux amorces d'ADN choisies de la manière indiquée ci-dessus,
- la mise en contact de l'échantillon biologique sus-mentionné avec une sonde nucléotidique telle que définie ci-dessus, dans des conditions permettant la production d'un complexe d'hybridation formé entre ladite sonde et ladite séquence de nucléotides,
- la détection du complexe d'hybridation sus-mentionné éventuellement formé.

A tire d'exemple de sondes nucléotidiques de l'invention, on citera la séquence nucléotidique de la figure 1, ou encore les séquences I et II de la figure 3.

L'invention ouvre enfin la voie à la mise au point de nouveaux principes vaccinants contre le paludisme issu de l'infection d'un individu par P. falciparum.

L'invention concerne également les compositions préparées sous forme de vaccins contenant soit le peptide selon l'invention, soit un oligomère de ce peptide, soit encore un conjugué de ce peptide ou oligomère avec une molécule porteuse, en association avec un véhicule pharmaceutiquement acceptable approprié et, le cas échéant, avec d'autres principes actifs vaccinants contre le paludisme.

Des compositions pharmaceutiques avantageuses sont constituées par des solutions, suspensions ou liposomes injectables contenant une dose efficace d'au moins un produit selon l'invention. De préférence, ces solutions, suspensions ou liposomes sont réalisés dans une phase aqueuse stérilisée isotonique, de préférence saline ou glucosée.

L'invention concerne plus particulièrement de telles suspensions, solutions ou forme liposome qui sont aptes à être administrées par injections intradermiques, intramusculaires ou sous-cutanées, ou encore par scarifications.

Elle concerne également des compositions pharmaceutiques administrables par d'autres voies, notamment par voie orale ou rectale, ou encore sous forme d'aérosols destinés à venir en contact avec des muqueuses, notamment les muqueuses oculaires, nasales, pulmonaires ou vaginales.

En conséquence, elle concerne des compositions pharmaceutiques dans lesquelles l'un au moins des produits selon l'invention se trouve associé à des excipients pharmaceutiquement acceptables, solides ou liquides, adaptés à la constitution de formes orales, oculaires ou nasales, ou avec des excipients adaptés à la constitution des formes d'administration rectale, ou encore avec des excipients gélatineux pour l'administration vaginale. Elle concerne aussi des compositions liquides isotoniques contenant l'un au moins des conjugués selon l'invention, adaptées à l'administration sur les muqueuses, notamment oculaires ou nasales.

Avantageusement, les compositions vaccinales selon l'invention contiennent en outre un véhicule, tel que la polyvinyl-pyrrolidone, facilitant l'administration du vaccin. A la place de la polyvinyl-pyrrolidone, on peut utiliser tout autre type d'adjuvant au sens classique que l'on donnait autrefois à cette expression, c'est-à-dire d'une substance permettant l'absorption plus aisée d'un médicament ou facilitant son action dans l'organisme. A titre d'exemples d'autres adjuvants de ce dernier type, on mentionnera encore la carboxyméthyl-cellulose, les hydroxydes et phosphates d'aluminium ou tous autres adjuvants de ce type, bien connus de l'homme de l'art. Enfin elles contiennent si besoin un adjuvant immunologique, notamment du type muramylpeptide.

L'invention ne se limite évidemment pas aux modes de réalisation décrits ci-dessus à titre d'exemples et l'homme de l'art peut y apporter des modifications sans pour autant sortir du cadre des revendications ci-après ; notamment certains des acides aminés intervenant dans la séquence des peptides selon l'invention peuvent être remplacés par des acides aminés isofonctionnels ou isostériques ; par exemple, une ou plusieurs des substitutions suivantes peuvent être envisagées :
- Glu est substitué par Asp ou Gln,
- Leu est remplacé par Ala, etc...

Il est naturellement bien entendu que les peptides qui résultent de telles substitutions consistent en des équivalents des peptides plus particulièrement revendiquées, dès lors qu'eux-mêmes ou des oligomères ou conjugués formés à partir de ces peptides présentent des propriétés immunogènes semblables.

L'invention concerne également encore plus particulièrement les "protéines chimères" qui peuvent être obtenues par les techniques du génie génétique, ces protéines chimères pouvant contenir une ou plusieurs séquences peptidiques, comportant respectivement les 87 acides aminés des séquences de l'invention, et incorporées ou rattachées à un fragment peptidique autre que la β-galactosidase. Ce dernier fragment peptidique a de préférence un poids moléculaire suffisant pour renforcer l'immunogénicité des séquences peptidiques selon l'invention et n'interfère pas du point de vue immunologique avec la manifestation de l'immunogénicité recherchée.

Des sérums provenant d'individus européens vivant dans des zones endémiques et suivant une prophylaxie continue avec des médicaments dirigés contre les schizontes des stades sanguins ont été sélectionnés et testés en utilisant des antigènes du stade des sporozoïtes (antigènes CS), du stade hépatique (antigène LSA), et des stades sanguins. La plupart de ces sérums réagissent avec les antigènes de tous les stades probablement car la prophylaxie a été interrompue. Trois sérums prélevés à partir d'individus ayant résidé en Afrique tropicale rurale et ayant ingéré 100 mg de chloroquine par jour sans interruption pendant 23 à 26 ans, ne réagissent pas avec les antigènes des stades sanguins suivant le test d'immunofluorescence (IFA) et le test d'immunotransfert sur nitro-cellulose. Ces trois sérums possedant toutefois des titres élevés en anticorps dirigés contre les sporozoïtes et les protéines de stade hépatique (dilution IFA 1/3200 et 1/6400 respectivement).

Un des trois sérums précédents , de spécificité réduite, a été utilisé pour cribler une banque d'ADN génomique construite dans le bactériophage λ gt 11 de la manière suivante :

### 1) CONSTRUCTION DE LA BANQUE D'ADN GENOMIQUE DE Plasmodium falciparum.

L'ADN génomique du clone 96 de la souche thaïlandaise Tak9 de P.falciparum (Science, 212, 1.37-1.38 (1981) a été isolé par les techniques classiques.

Des échantillons de 18 »g d'ADN de P.falciparum ont été incubés à 15°C dans un tampon 50 mM Tris HCl pH 7.5, 1 mM MnCl₂, 20 »g/ml de sérum albumine bovine, avec des quantités respectives d'ADNase I (Boehringer Mannheim) de 5 pg pendant 5 minutes ou bien de 3.5 pg pendant 5 ou 10 minutes. Après addition de 5 mM EDTA (Ethylènediamine tetracétique acide), les échantillons d'ADN sont réunis et purifiés par extraction au mélange phénol/chloroforme/alcool isoamylique (25 V/24 V/1 V) puis par l'éther. L'ADN est concentré par précipitation à l'éthanol à -20°C en présence de 2.5 M d'acétate d'ammonium.

45 »g d'ADN ainsi traité ont été méthylés par 180 U d'Eco R1 méthylase (Biolabs) dans les conditions recommandées par le fournisseur, avec l'addition supplémentaire de 5 mM DTT (dithiothreitol), pendant 15 minutes à 37° C. Après purification de l'ADN comme ci-dessus, 10 »g d'ADN ont été incubés avec 40 mM Tris HCl pH 8.0, 10 mM sulfate d'ammonium, 10 mM 2-mercaptoethanol, 0.5 mM EDTA, 0.05 mM NAD (nicotinamide adénine dinucléotide) 0.1 mM dXTP (comprenant les 4 desoxynucleotides triphosphates dATP, dCTP, dGTP et dTTP), en présence de 10 U T4 ADN polymérase (PL Biochemicals) et de 10 U de E. coli ADN ligase (Biolabs). L'ADN a été purifié et concentré comme ci-dessus.

8 »g d'ADN ont alors été ligaturés avec 0.4 »g d'un adaptateur ou "linker" Eco R1 (adaptateurs phosphorylés Eco R1 commercialisés par Biolabs) par 4 U T4 ADN ligase (Biotec) dans le tampon 50 mM Tris HCl pH 8.0, 10 mM MgCl₂, 20 mM DTT, 1 mM ATP, 50 »g/ml sérum albumine bovine.

Après incubation à 4° C pendant 5 heures, on ajoute 2 U T4 ADN ligase et la réaction est poursuivie à 4° C pendant 16 heures. Le tube est soumis à plusieurs cycles de congélation à -80° C / décongélation pour arrêter la réaction. L'ADN est ensuite dilué et le tampon d'incubation ajusté de façon à obtenir les conditions recommandées par le fournisseur pour l'utilisation de l'enzyme Eco R1. 100 U d'enzyme Eco R1 (Promega Biotec) sont ajoutées et incubées pendant 3 heures à 37°C. La réaction est arrêtée par un chauffage de 10 minutes à 60° C, et l'ADN est purifié et concentré comme ci-dessus.

L'ADN est resuspendu dans 100 »l de tampon 50 mM Tris HCl pH 8.0, 1 mM EDTA, et déposé sur un gradient 5-20 % de saccharose préparé en 25 mM acétate de sodium, 10 mM EDTA et centrifugé dans le rotor Beckman SW 50.1 à 45,OOO tours par minute pendant 150 minutes. Les fractions sont analysées sur gel d'agarose et celles qui contiennent les fragments d'ADN de tailles comprises entre environ 300 pb. et 2 500 pb. sont rassemblées, dialysées contre le tampon 50 mM Tris HCl pH 8.0, 1 mM EDTA à 4°C. L'ADN est concentré par précipitation à l'éthanol. Environ 400 ng de cet ADN ont été ligaturés à 1 »g d'ADN du vecteur gt11 (Proc. Natl. Acad. Sci., USA, 80, 1194-1198 (1983)) coupé par Eco R1 et déphosphorylé (Protoclone de Promega Biotec), dans un volume de 10»l, (en tampon 50 mM Tris HCl pH 8.0, 10 mM MgCl₂, 20 mM DTT, 1 mM ATP, 50 »g/ml sérum albumine bovine) par 1 U T4 ADN ligase (Biotec).

Les produits de ligature ont été encapsidés in vitro dans les extraits d'E. coli préparés à partir des souches bactériennes construites par B. Hohn (Methods Enzymol. 68, 299), selon la technique décrite par Maniatis et coll. (Molecular cloning, a laboratory manual, p. 264, Cold Spring Harbor Laboratory (1982)).

Environ 7 millions de bactériophages recombinants ont été obtenus.

### 2) CRIBLAGE IMMUNOLOGIQUE DE LA BANQUE

Les bactériophages recombinants ont été étalés sur un milieu de culture contenant la bactérie indicatrice Y 1090, à une densité de 50,000 plages par boîte de Pétri de 90 mm, et incubés à 42°C pendant 3 heures.

Un filtre de nitrocellulose (Schleicher & Schuell, BA 85) saturé par 0,01 M IPTG isopropyl-b-thiogalactopyranoside (Sigma) est déposé sur les boîtes, qui sont incubées à 37°C pendant 3 heures. Au terme de ces incubations, les filtres de nitrocellulose sont prélevés, et les boîtes de Pétri conservées à 4° C.

Les filtres de nitrocellulose sont placés dans un bain de tampon TL : 50 mM tris HCl pH 8.0, 150 mM NaCl, 5 % lait écrémé, O.05 % Tween 20 (Sigma). Les filtres sont incubés 15 heures à 4° C en tampon TL, puis 2 fois 15 minutes à 20° C. Ils sont alors incubés pendant une heure avec un pool d'antisérums humains immuns dirigés contre les antigènes de tous les stades de développement de P. falciparum, traité au préalable pour le dépléter en anticorps anti-E.coli selon la technique décrite par Ozaki et coll. (J. Immunol. Methods, 89, 213-219, 1986). Le pool d'antisérums humains a été utilisé à la dilution 1/200, en tampon TL. L'incubation a été faite à 20°C pendant 1 heure. Les filtres ont été lavés 4 fois avec le tampon TL, puis incubés avec des anticorps anti-immunoglobulines humaines conjugués à la peroxydase de raifort (Biosys) et iodinés à l'iode ¹²⁵I, pendant 1 heure à 20° C. Après plusieurs lavages en tampon TL, puis en tampon 50 mM Tris HCl pH 8.0, 150 mM NaCl, l'activité enzymatique de la peroxydase est révélée (Ozaki et coll, précédemment cité), les filtres sont séchés à l'air libre et autoradiographiés sur film Kodak Royal X-OMat AR, avec un écran amplificateur.

Une collection d'environ 1 200 clones de bactériophages recombinants a été constituée en prélevant les plages de lyse correspondant aux signaux positifs. Ces clones ont été ensuite soumis à un second cycle de criblage immunologique, en employant cette fois un des trois sérums humains précédemment décrits et présentant pas ou peu d'anticorps dirigés contre les formes érythrocytaires de P. falciparum et un titre élevé contre les formes hépatiques du parasite. Ce criblage immunologique a été effectué selon le protocole décrit ci-dessus. Ce sérum réagit avec seulement 15 % des clones producteurs d'un antigène spécifique de P.falciparum (120 sur 1200 testés) et parmi les clones les plus actifs, 60 ont été sélectionnés et étudiés comme suit.

Les anticorps humains qui réagissent avec les déterminants antigéniques exprimés par les clones recombinants ont été purifiés par affinité sur les protéines recombinantes, selon la technique décrite par Ozaki et coll. (précédemment cité). Ces anticorps spécifiques ont été incubés avec des préparations de parasites à différents stades de développement (sporozoïte, stade hépatique ou stades érythrocytaires), et la réaction a été étudiée par immunofluorescence indirecte, et immunotransfert. Le clone recombinant sur lequel sont retenus par affinité des anticorps spécifiques du stade hépatique, et du stade sporozoïte et donc qui expriment des déterminants propres à ces stades ont été étudiés : il s'agit du clone DG 671. Ces anticorps spécifiques de ce clone réagissent spécifiquement
a) d'une part avec les schizontes hépatiques, tels que l'on peut les obtenir après infection d'hépatocytes humains ou de singes par des sporozoïtes de P.falciparum ; la localisation de la fluorescence a été déterminée comme étant identique à celle considérée caractéristique des antigènes de stade hépatique, ;
b) d'autre part, avec la surface des sporozoïtes de la soude plasmodiale NF54, mais non celle du clone parasitaire 3D7, et avec celle de 2 des 8 isolats d'origine thaïlandaise testés. Cette réactivité avec les antigènes de surface est mise en évidence dans le test d'IFI employant des sporozoïtes dits "humides" ou en suspension, qui sont attachés à une lame de verre par l'intermédiaire d'un film de Poly-L-lysine selon la technique d'IFI décrite par DRUILHE et al (Infection and Immunity, (1986) 53, 393-397). La localisation de l'antigène avec lequel les anticorps réagissent en surface des sporozoïtes a, de plus, été confirmée au niveau ultra-structural en révélant la fixation des anticorps par un deuxième anticorps anti-immuno- globulines humaines couplé à des grains d'or colloïdal. Par ailleurs, dans un test d'IFI employant des sporozoïtes séchés et fixés par l'acétone, ces anticorps réagissent aussi bien avec les sporozoïtes de la souche NF54, que du clone parasitaire 3d7. Enfin, dans un test d'immunotransfert (Immunoblotting) en utilisant des protéines extraites en SDS des stades sporozoïtes, ces anticorps révèlent un polypeptide de poids moléculaire 70 kilodaltons dans la souche NF54, le clone parasitaire 3D7 et l'une des deux souches thaïlandaises positives testées. Par contre, aucun polypeptide n'est révélé dans les mêmes conditions dans les extraits de sporozoïtes de souches avec lesquelles ce test d'IFI était négatif. Il ressort de ces considérations que l'antigène révélé par ces anticorps peut être soit présent de façon interne, c'est-à-dire dans le cytoplasme du stade sporozoïte, mais non en surface, soit à la fois de façon interne et aussi en surface, soit non présent.

En conséquence des caractéristiques a) et b) ci-dessus, l'antigène codé par la séquence nucléique du clone DG671 est dénommé "SALSA" (Sporozoïte and Liver Stage Antigen).

La spécificité d'espèce et de stade du clone DG671 a été testée de la manière suivante. Premièrement, il a été déterminé que les mêmes anticorps purifiés par affinité et qui réagissent par IFA (ou encore qui sont IFA positifs) avec les antigènes de stade hépatique et les sporozoïtes, ne réagissent pas avec les antigènes des stades sanguins, qu'ils soient testés par IFA avec des parasites fixés à l'acétone, ou par immunotransfert (immunoblotting) en utilisant des protéines extraites au SDS. Les anticorps purifiés par affinité ne réagissent pas avec les antigènes de stade sporozoïte de P. yoelii, de P. berghei et de P.vivax ni avec ces antigènes de stade hépatique de P. yoelii, ni avec les schizontes hépatiques de P. vivax préparés à partir de singes Saimiri sciureus.

Deuxièmement, les protéines recombinantes de DG 671 ne réagissent pas avec les sérums provenant de deux patients atteints de malaria (paludisme causé par P.falciparum) par transfusion accidentelle et qui, par définition n'ont donc pas d'anticorps contre les antigènes spécifiques des stades précédents (sporozoïtes et antigènes du stade hépatique). Ces protéines ne réagissent pas avec deux anticorps monoclonaux reconnaissant le tétrapeptide CS, avec les sérums de souris immunisées avec les antigènes CS recombinants R₃₂têt32 (YOUNG J.F. et al, Science, 228, 957-958 (1986)), ni avec des sérums de lapins immunisés avec les régions I et II de la protéine CS. De plus, les protéines recombinantes n'ont pas réagi avec des antisérums humains dirigés contre P. vivax (bien que les sérums furent positifs avec les schizontes hépatiques de P. vivax), P. ovale et P. cynomolgi (Ann. Soc. Belg. Med. Trop., 60, 348 (1980)) quand elles sont testées par la technique de taches d'immunotransfert (immunodot blots), alors qu'elles sont positives avec tous les sérums humains anti-P. falciparum testés.

L'insérat de 261 paires de bases de P. falciparum a été purifié et recloné à l'intérieur du plasmide pUC13 (Nucleic Acids Research, 9, 309-321 (1981)) . La séquence d'ADN et l'organisation génomique du gène SALSA ont alors été déterminées. La figure 1 montre que le clone DG 671 contient un fragment d'ADN composé entièrement d'un motif de 261 paires de bases sans répétition.

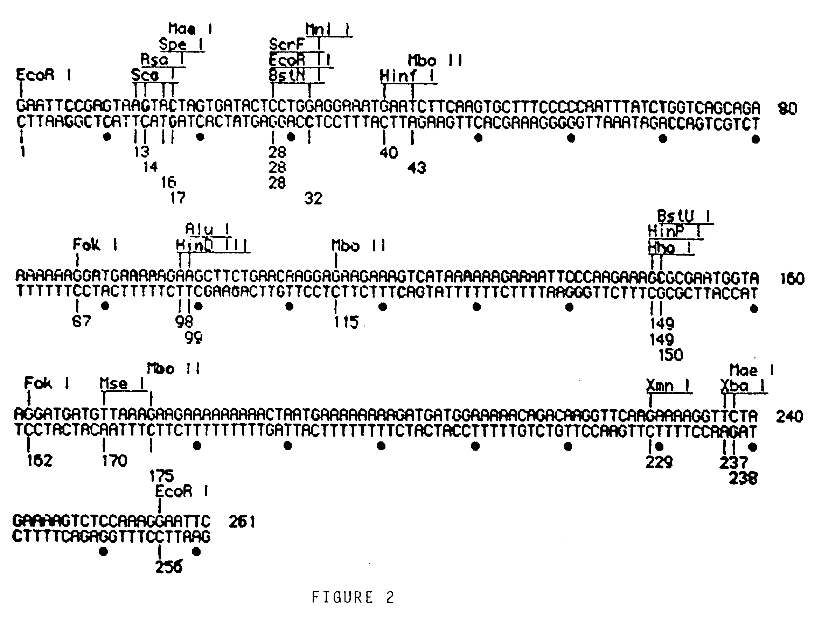

Seulement une phase de lecture est en phase avec le gène lacZ de la β-galactosidase, une caractéristique attendue puisque le clone produit une protéine de fusion qui porte les épitopes reconnus par le sérum humain. La séquence en acides aminés correspondant à ce fragment est représentée à la figure 2. Elle est composée d'une chaîne de 87 acides aminés riche en acide glutamique, lysine et serine mais ne contient ni methionine, ni cystéine. L'analyse informatique de cette séquence indique qu'elle a une grande probabilité de posséder une structure en hélice au niveau des acides aminés comris entre les positions 10 à 50. De plus, aucune homologie entre les séquences d'ADN de P.falciparum connues à l'heure actuelle et la séquence d'ADN codant pour ladite protéine n'a été détectée par analyse des banques de données Los Alamos et NBRF. Par contre, il existe entre la séquence d'ADN codant pour ladite protéine SALSA et la séquence d'un ADNc (ADN complémentaire) d'origine hépatique humaine une homologie à 60,2 %. Cette homologie au niveau nucléotidique ne se traduit pas par une homologie réelle au niveau peptidique (en effet, ce degré d'homologie n'est alors que de 20 à 30 %). Ceci est en accord avec la spécificité de stade hépatique et de stade sporozoïte de la protéine SALSA.

Des analyses par transfert selon la méthode de Southern utilisant le fragment de 261 paires de bases cloné dans pUC 13 indiquent que ce fragment s'hybride à 2 fragments Eco RI. Il est présent dans les 2 souches de P. falciparum examinées jusqu'à maintenant, localisé sur le chromosome n°2. Cette localisation permet de conclure que le gène correspondant est distinct de celui de la proteine CS des sporozoïtes et distinct de celui de l'antigène LSA précédemment décrit.

Les études d'hybridation d'ADN du clone DG671 avec le sous-ensemble de 120 clones d'ADN décrit précédemment, et codant vraisemblablement pour des antigènes de stade pré-érythrocytaire, a révélé que la structure du clone DG671 était unique dans ce sous-ensemble : l'ADN de DG671 n'hybride qu'avec lui-même et n'hybride pas avec l'ADN des 119 autres clones.

Comme les épitopes répétitifs apparaissent être une caractéristique fréquente des antigènes de P. falciparum (Nature, 306, 751-756 (1983) ; Nature, 311, 382-385 (1984) ; Science, 225, 593-599 (1984) ; Science, 227, 1595-1597 (1985) ; Cell, 40, 775-783 (1985)), il est important de souligner que le polypeptide SALSA, bien qu'il soit hautement immunogène chez l'homme ne possède pas de structure répétitive.

La grande prévalence d'anticorps contre l'antigène SALSA est une de ses caractéristiques importantes. La présence d'anticorps chez les individus exposés au paludisme et provenant de 3 régions d'Afrique différentes par le niveau de transmission de la maladie, a été étudiée en immunotransfert sur nitro-cellulose des protéines recombinantes produites dans des colibacilles après séparation par électrophorèse. Une prévalence variant de 90 à 95 % des individus étudiés selon la zone, a été observée. Ces résultats sont particulièrement significatifs si on les compare aux résultats obtenus avec d'autres antigènes de P.falciparum. Ainsi, dans la zone de plus faible transmission, (le plus faible nombre de piqûres infectieuses) la prévalence d'anticorps contre la protéine CS (déterminée par ELISA avec le peptide R₃₂têt32) n'est que de 27 % contre 65 % pour les antigènes des stades sanguins (déterminé par IFI sur frottis de globules rouges parasités), ou contre 75 % pour l'antigène LSA (déterminé par immunotransfert), ou contre 81 % pour le LSA synthétique (déterminé par ELISA avec le peptide synthétique de 41 acides aminés contenant 2,5 répétitions de 17 acides aminés),ou contre 97 % pour le SALSA (en immunotransfert). Le tableau I, ci-après, représente la comparaison des résultats obtenus dans une expérience du même type, en Immunotransfert avec l'antigène DG671 SALSA et l'antigène LSA (WB SALSA, WB LSA), par rapport à ceux obtenus en ELISA avec la tétrapeptide répétitif de la circumsporozoïte protéine (ELISA NANP), et en immunofluorescence avec des schizontes hépatiques de P. falciparum (IFA,LS, LS correspondant à l'abbréviation de "Liver Stage" soit stage hépatique).

**TALBEAU I**

| ZONE DE FAIBLE ENDEMIE | | | | |
|---|---|---|---|---|
| AGE | ELISA NANP | IFA LS | W.B. LSA | W.B. SALSA |
| 0-10 | 0/12 | 12/12 | 8/12 | 11/12 |
| 10-20 | 0/5 | 5/5 | 4/5 | 5/5 |
| > 20 | 7/13 | 13/13 | 12/13 | 11/13 |
| total | 7/30 | 30/30 | 24/30 | 27/30 |

Au vu de ces résultats et de la présence inconstante des épitopes contenus dans la protéine recombinante SALSA dans les sporozoïtes de divers isolats, on peut émettre l'hypothèse que les épitopes sont plus fréquemment exprimés au niveau du stade hépatique que du stade sporozoïte.

L'analyse de la conformation de la séquence d'acides aminés selon la technique de Chou et Fassman (Prediction of Protein Conformation, Biochemistry, vol. 13, n°2, 222-245 (1974) permet de prédire que 3 zones de la molécule SALSA ont une forte tendance à s'organiser en hélice α, l'une comprise entre les acides aminés 25 à 51, les deux autres comprises entre les acides aminés 52 à 87. En conséquence, des peptides synthétiques représentant les séquences suivantes ont été préparés:
1 - SAEKKDEKEASEQGEESHKKENSQESA dénommé SALSA1,
2- NGKDDVKEEKKTNEKKDDGKTDKVQEKVLEKSPKEF dénommé SALSA2.

Ils ont été purifiés et utilisés dans divers tests immunologiques.

En ELISA, des sérums de sujets vivant en zone de faible endémie réagissent spécifiquement avec chacun de ces peptides. Chez trois groupes d'individus, appartenant à toutes les classes d'age, la prévalence d'anticorps anti-SALSA 1 ou 2 varie de 25 % à 60 %. Il existe un parallélisme non strict des réponses vis-à-vis de la protéine recombinante DG671. Une étude de la re-positivation du sang (réapparition des parasites), après une cure radicale du paludisme, et des réponses aux peptides SALSA 1 et 2, a été realisée de la manière suivante : un groupe de 250 individus a reçu une cure radicale de chloroquine (dose = 25 mg/kg, suffisante pour éradiquer tous les parasites présents) dans le but de supprimer toute parasitémie pré-existante. Ils ont ensuite été suivis cliniquement et parasitologiquement pendant 3 mois de la saison de transmission de paludisme par l'anophèle pour détecter la ré-émergence du parasite dans le sang. Deux groupes de 40 individus chacun ont été sélectionnés, les uns n'ayant pas présenté de parasitémie pendant la durée du suivi, les autres ayant un examen de sang positif pour P. falciparum (frottis et goutte épaisse colorés au giemsa). Chez les sujets dont l'examen de sang est resté négatif, la prévalence et les titres moyens d'anticorps vis-à-vis des peptides SALSA 1 et 2, sont significativement plus élevés que chez ceux ayant présenté une parasitémie. Par contre, la prévalence de répondeurs et les taux d'anticorps vis-à-vis d'un antigène témoin, le tétrapeptide répétitif NANP de la circumsporozoïte protéine sont identiques dans les deux groupes. Ces résultats suggèrent que la réponse immunitaire au DG 671-SALSA interfère avec le déroulement de la phase de multiplication intra-hépatique du parasite. Par contre, il n'y a aucune indication que la réponse à la circumsporozoïte protéine puisse avoir le même effet. En d'autres termes, ces résultats suggérent que le développement d'une immunité contre la molécule SALSA peut avoir un effet protecteur chez l'homme contre l'infection à Plasmodium falciparum.

La réponse proliférative des lymphocytes de sujets exposés au paludisme a été étudiée vis-à-vis de l'antigène peptidique SALSA1. Les résultats montrent que cette séquence peptidique comporte une épitope reconnu par les lymphocytes T d'une proportion importante de sujets exposés.

Les lymphocytes CD4⁺ de 7 des 10 sujets étudiés ont proliféré en présence du peptide SALSA1 (1 et 10 »g/ml) et en présence d'interleukine-2 (IL-2). Le ratio de prolifération par rapport au témoin IL-2 seul est supérieur à 10. Ce résultat montre que les sujets génétiquement capables de répondre au peptide SALSA1 sont plus fréquemment rencontrés en zone endémique que ceux capables de répondre aux épitopes T de la circumsporozoïte. En d'autres termes, les épitopes T du polypeptide SALSA sont moins génétiquement restreints que ceux de la circumsporozoïte protéine.

L'invention concerne encore les acides nucléiques recombinants contenant la séquence codant pour le polypeptide SALSA, ainsi que les micro-organismes, notamment les bactéries E.coli tranformées par ces acides nucléiques recombinants et capables d'exprimer ledit polypeptide.

L'invention concerne ces séquences d'acides nucléiques ou des séquences équivalentes qui peuvent être synthétisées et qui codent pour les mêmes acides aminés.

Il apparaîtra immédiatement à l'homme de métier que dans ces séquences, certains des nucléotides peuvent être remplacés par d'autres en raison de la dégénéréscence du code génétique sans que pour autant les peptides codés ne soient modifiés. Toutes ces séquences nucléotidiques, ainsi que celles qui codent pour des polypeptides qui différent des précédents par un ou plusieurs acides aminés sans que leur activité immunogénique propre ne soit modifiée de façon semblable, font partie de l'invention. Il en va naturellement de même des séquences nucléotidiques qui peuvent être reconstituées et qui sont capables de coder pour des oligomères tels qu'ils ont été définis plus haut. Les motifs monomères sont liés directement bout à bout ou par l'intermédiaire de séquences peptidiques sans effet sur les propriétés immunogéniques des oligomères ainsi formés.

Enfin, l'invention concerne les vecteurs modifiés par ces micro-organismes, ces vecteurs étant naturellement pourvus d'éléments de régulation et de terminaison précédant et suivant les séquences nucléiques sus-indiquées, qui permettront l'expression de ces dernières dans des organismes cellulaires compétents.

Des bactéries hébergeant le susdit clone DG 671 ont été déposées à la Collection Nationale des Cultures de Microorganismes de l'Institut Pasteur de Paris (CNCM), le 31 mars 1989 sous le numéro I-855.

## Revendications

1. Polypeptide tel que produit par la souche bactérienne transformée par le plasmide DG-671 et déposée à la CNCM sous le n° I-855 le 31 mars 1989.

2. Polypeptide selon la revendication 1, caractérisé en ce qu'il est constitué d'un peptide monomère.

3. Polypeptide selon la revendication 1, représenté par tout ou partie de l'enchaînement d'acides aminés suivant :

Asn-Gly-Lys-Asp-Asp-Val-Lys-Glu-Glu-Lys-Lys-Thr-Asn-
Glu-Lys-Lys-Asp-Asp-Gly-Lys-Thr-Asp-Lys-Val-Gln-Glu-
Lys-Val-Leu-Glu-Lys-ser-Pro-Lys-Glu-Phe.

4. Polypeptide caractérisé en ce qu'il est codé par l'acide nucléique contenu dans le plasmide DG671 utilisé pour transformer la souche bactérienne déposée à la CNCM sous le n° 1-855 le 31 mars 1989.

5. Séquence de nucléotidique caractérisée en ce qu'elle comprend un enchaînement de nucléotides codant pour des polypeptides selon l'une des revendications 1 à 4.

6. Séquence de nucléotides, contenu dans le plasmide DG671 utilisé pour transformer la souche bactérienne déposée à la CNCM sous le n° I-855 le 31 mars 1989.

7. Séquence de nucléotides capable de s'hybrider avec la séquence nucléotidique de la revendication 6, ou la séquence complémentaire de cette dernière dans les conditions suivantes :
- pré-traitement (pré-hybridation) du filtre de nitrocellulose supportant le fragment d'acide nucléique à tester avec le tampon d'hybridation, (composé de 6 x SSC, 2,5 % de lait, 0,5 % de SDS), cette opération étant effectuée à 65° C pendant 1 heure ;
- remplacement du tampon d'hybridation au contact du support, sur lequel le fragment d'acide nucléique est alors fixé, par du tampon d'hybridation de même composition (comprenant en outre de l'ADN de saumon dénaturé), et addition de la séquence de la revendication 6 en tant que sonde, notamment marquée radioactivement, et préalablement dénaturée par un traitement à 100°C pendant 5 minutes ;
- incubation dudit fragment d'acide nucléique fixé sur le support dans ce tampon d'incubation avec la séquence de la figure 1 à 65° C pendant une durée de 16 à 24 heures,
- l'élimination du tampon contenant la sonde non fixée, par 3 lavages successifs de 5 minutes chacun avec 2 x SSC, 0,1 % SDS à 65°C, suivis de 3 lavages successifs de 5 minutes chacun avec 0,1 x SSC, 0,1 % SDS à 65°C.

8. Amorce d'ADN ou d'ARN, caractérisée en ce qu'elle est constituée d'environ 10 à 25 nucléotides, identiques aux 10 à 25 premiers nucléotides de la séquence de nucléotides codant pour une séquence peptidique selon l'une des revendications 1 à 4, ou identiques aux 10 à 25 derniers nucléotides de ladite séquence.

9. Amorce d'ADN ou d'ARN, caractérisée en ce qu'elle est constituée d'environ 10 à 25 nucléotides complémentaires de 10 à 25 premiers nucléotides de la séquence nucléotidique codant pour une séquence peptidique selon l'une des revendications 1 à 4, ou complémentaire des 10 à 25 derniers nucléotides de ladite séquence de nucléotides.

10. Amorce d'ADN ou d'ARN selon la revendication 9, caractérisée en ce qu'elle est constituée d'environ 10 à 25 nucléotides capables de s'hybrider avec les 10 à 25 premiers nucléotides ou avec les 10 à 25 derniers nucléotides de ladite séquence de nucléotides codant pour une séquence peptidique selon l'une des revendications 1 à 4, dans les conditions d'hybridation définies dans la revendication 7.

11. Acide nucléique recombinant, caractérisé en ce qu'il contient au moins une séquence de nucléotides selon l'une des revendications 5 à 7, insérée dans un acide nucléique hétérologue vis-à-vis de la susdite séquence.

12. Acide nucléique recombinant selon la revendication 11, caractérisé en ce que la séquence de nucléotides selon l'une des revendications 5 à 7, est précédée d'un promoteur sous le contrôle duquel la transcription de ladite séquence est susceptible d'être effectuée et, le cas échéant, suivie d'une séquence codant pour des signaux de terminaison de la transcription.

13. Vecteur recombinant, en particulier pour le clonage et/ou l'expression, notamment du type plasmidique, cosmide ou phage, caractérisé en ce qu'il contient un acide nucléique recombinant selon la revendication 11 ou la revendication 12, en l'un de ses sites non essentiels pour sa réplication.

14. Vecteur recombinant selon la revendication 13, caractérisé en ce qu'il s'agit du plasmide DG-671 déposé à la CNCM sous le n° I-855 le 31 mars 1989.

15. Vecteur recombinant selon la revendication 13, caractérisé en ce qu'il contient en l'un de ses sites non essentiels pour sa réplication, des éléments nécessaires pour promouvoir l'expression d'une séquence peptidique selon l'une des revendications 1 à 4 dans un hôte cellulaire, et éventuellement un promoteur reconnu par les polymérases de l'hôte cellulaire, en particulier un promoteur inductible.

16. Hôte cellulaire transformé par un vecteur recombinant selon l'une quelconque des revendications 13 à 15 et comprenant les éléments de régulation permettant l'expression de la séquence de nucléotides codant pour un polypeptide selon l'une des revendications 1 à 4 dans cet hôte.

17. Procédé de préparation d'un polypeptide selon l'une des revendications 1 à 4, comprenant les étapes suivantes ;
- le cas échéant, l'amplification préalable suivant la technique PCR de la quantité de séquences de nucléotides codant pour ledit polypeptide à l'aide d'une amorce d'ADN selon la revendication 8, et d'une amorce d'ADN selon la revendication 9 ou la revendication 10 choisies de manière à ce que l'une de ces amorces soit identique aux 10 à 25 premiers nucléotides de la séquence nucléotidique codant pour ledit polypeptide, tandis que l'autre amorce est complémentaire des 10 à 25 derniers nucléotides (ou s'hybride avec ces 10 à 25 derniers nucléotides) de ladite séquence nucléotidique, ou inversement de manière à ce que l'une de ces amorces soit identique aux 10 à 25 derniers nucléotides de ladite séquence, tandis que l'autre amorce est complémentaire des 10 à 25 premiers nucléotides (ou s'hybride avec les 10 à 25 premiers nucléotides) de ladite séquence nucléotidique, suivie de l'introduction desdites séquences de nucléotides ainsi amplifiées dans un vecteur approprié,
- la mise en culture, dans un milieu de culture approprié, d'un hôte cellulaire préalablement transformé par un vecteur approprié contenant un acide nucléique selon la revendication 11 ou 12, et
- la récupération, à partir du susdit milieu de culture du polypeptide produit par ledit hôte cellulaire transformé.

18. Sonde nucléotidique de détection caractérisée en ce qu'elle est constituée par tout ou partie d'une séquence de nucléotides selon l'une des revendications 5 à 7.

19. Méthode de diagnostic in vitro du paludisme chez un individu susceptible d'être infecté par P. falciparum qui comprend les étapes suivants :
- éventuellement l'amplification préalable de la quantité de séquences de nucléotides selon l'une quelconque des revendications 5 à 7, susceptibles d'être contenues dans l'échantillon biologique prélevé chez ledit individu, à l'aide d'une amorce d'ADN selon la revendication 8 et d'une amorce d'ADN selon la revendication 9 ou 10, et choisies de la manière indiquée dans la revendication 17,
- la mise en contact de l'échantillon biologique susmentionné avec une sonde nucléotidique selon la revendication 18, dans des conditions permettant la production d'un complexe d'hybridation formé entre ladite sonde et ladite séquence de nucléotides,
- la détection du complexe d'hybridation sus-mentionné éventuellement formé.

20. Méthode de diagnostic in vitro du paludisme chez un individu susceptible d'être infecté par P. falciparum qui comprend la mise en contact d'un tissu ou d'un fluide biologique prélevé chez un individu avec un polypeptide selon l'une des revendications 1 à 4 dans des conditions permettant une réaction immunologique in vitro entre ledit polypeptide et les anticorps éventuellement présents dans le tissu biologique, et la détection in vitro du complexe antigène-anticorps éventuellement formé.

21. Nécessaire ou kit pour la mise en oeuvre d'une méthode de diagnostic in vitro selon la revendication 19, caractérisé en ce qu'il comprend :
- une quantité déterminée d'une sonde nucléotidique selon la revendication 18,
- avantageusement un milieu approprié à la formation d'une réaction d'hybridation entre la séquence à détecter, et la sonde sus-mentionnée,
- avantageusement des réactifs permettant la détection des complexes d'hybridation formés entre la séquence de nucléotides et la sonde lors de la réaction d'hybridation.

22. Nécessaire ou kit pour le diagnostic in vitro du paludisme chez un individu susceptible d'être infecté par P.falciparum selon la revendication 20 qui comprend :
- un polypeptide selon l'une des revendications 1 à 4,
- les réactifs pour la constitution du milieu propice à la réalisation de la réaction immunologique,
- les réactifs permettant la détection du complexe antigènes-anticorps produit par la réaction immunologique, de tels réactifs pouvant également porter un marqueur, ou être susceptibles d'être reconnus à leur tour par un réactif marqué, plus particulièrement dans le cas où le polypeptide sus-mentionné n'est pas marqué.

23. Les anticorps, polyclonaux ou monoclonaux, qui reconnaissent spécifiquement la séquence peptidique selon l'une des revendications 1 à 4.

24. Composition immunogène caractérisée par l'association d'un polypeptide conforme à l'une des revendications 1 à 4, en association avec un véhicule pharmaceutiquement acceptable.

25. Composition de vaccin dirigée contre le paludisme, contenant entre autres principes immunogènes, un polypeptide conforme à l'une des revendications 1 à 4.

## Claims

1. Polypeptide such as that produced by the bacterial strain transformed by the plasmid DG-671 and deposited with the CNCM under No.I-855 on 31 March 1989.

2. Polypeptide according to Claim 1, characterized in that it is constituted by a monomeric peptide.

3. Polypeptide according to Claim 1, represented by all or part of the following amino acid sequence :

ASn-Gly-Lys-Asp-Asp-Val-Lys-Glu-Glu-Lys-Lys-Thr-Asn-
Glu-Lys-Lys-Asp-Asp-Gly-Lys-Thr-Asp-Lys-Val-Gln-Glu-
Lys-Val-Leu-Glu-Lys-ser-Pro-Lys-Glu-Phe.

4. Polypeptide characterized in that it is coded by the nucleic acid contained in the plasmid DG-671 used to transform the bacterial strain deposited with the CNCM under the No. I-855 on 31 March 1989.

5. Nucleotide sequence characterized in that it comprises a nucleotide sequence coding for polypeptides according to one of the Claims 1 to 4.

6. Nucleotide sequence contained in the plasmid DG-671 used to transform the bacterial strain deposited with the CNCM under the No. I-855 on 31 March 1989.

7. Nucleotide sequence capable of hybridizing with the nucleotide sequence of Claim 6, or the sequence complementary to this latter under the following conditions :
- pre-treatment (pre-hybridization) of the nitrocellulose filter used as support for the test nucleic acid fragment with the hybridization buffer (composed of 6 x SSC, 2.5 % milk, 0.5 % SDS), this operation being carried out at 65° C for 1 hour;
- replacement of the hybridization buffer in contact with the support to which the nucleic acid fragment is then bound by the hybridization buffer of the same composition (containing in addition denatured salmon DNA) and addition of the sequence of Claim 6 as probe, in particular radioactively labelled, and denatured beforehand by a treatment at 100°C for 5 minutes ;
- incubation of the said nucleic acid fragment bound to the support in this incubation buffer with the sequence shown in Figure 1 at 65°C for 16 to 24 hours,
- removal of the buffer containing the unbound probe by 3 successive washings of 5 minutes each with 2 x SSC, 0.1 % SDS at 65°C, followed by 3 successive washings of 5 minutes each with 0.1 x SSC, 0.1 % SDS at 65°C.

8. DNA or RNA primer, characterized in that it is constituted of about 10 to 25 nucleotides, identical with the first 10 to 25 nucleotides of the nucleotide sequence coding for a peptide sequence according to one of the Claims 1 to 4, or identical with the last 10 to 25 nucleotides of the said sequence.

9. DNA or RNA primer, characterized in that it is constituted of about 10 to 25 nucleotides complementary to the first 10 to 25 nucleotides of the nucleotide sequence coding for a peptide sequence according to one of the Claims 1 to 4, or complementary to the last 10 to 25 nucleotides of the said sequence.

10. DNA or RNA primer according to Claim 9, characterized in that it is constituted of about 10 to 25 nucleotides capable of hybridizing with the first 10 to 25 nucleotides or with the last 10 to 25 nucleotides of said nucleotide sequence coding for a peptide sequence according to one of the Claims 1 to 4, under the conditions of hybridization defined in Claim 7.

11. Recombinant nucleic acid, characterized in that it contains at least one nucleotide sequence according to one of the Claims 5 to 7, inserted in a nucleic acid heterologous with respect to the above-mentioned sequence.

12. Recombinant nucleic acid according to Claim 11, characterized in that the nucleotide sequence according to one of the Claims 5 to 7 is preceded by a promoter under the control of which the transcription of the said sequence is capable of being carried out and, optionally, followed by a sequence coding for termination signals of transcription.

13. Recombinant vector, particularly of the plasmid, cosmid or phage type, in particular for the cloning and/or the expression, characterized in that it contains a recombinant nucleic acid according to Claim 11 or Claim 12 at one of its site inessential for its replication.

14. Recombinant vector according to Claim 13, characterized in that it is the plasmid DG-671 deposited with the CNCM under the No. I-855 on 31 March 1989.

15. Recombinant vector according to Claim 13, characterized in that it contains at one of its sites inessential for its replication elements necessary to promote the expression of a peptide sequence according to one of the Claims 1 to 4 in a cell host, and optionally a promoter recognized by the polymerases of the cell host, in particular an inducible promoter.

16. Cell host transformed by a recombinant vector according to any one of the Claims 13 to 15 and comprising the regulatory elements permitting the expression of the nucleotide sequence coding for a polypeptide according to one of the Claims 1 to 4 in this host.

17. Procedure for the preparation of a polypeptide according to any one of the Claims 1 to 4, comprising the following steps :
- optionally, the prior amplification according to the PCR technique of the quantity of nucleotide sequences coding for the said polypeptide with the aid of a DNA primer according to Claim 8, and a DNA primer according to Claim 9 or Claim 10 selected so that one of these primers is identical with the first 10 to 25 nucleotides of the nucleotide sequence coding for the said polypeptide, whereas the other primer is complementary to the last 10 to 25 nucleotides (or hybridizes with these last 10 to 25 nucleotides) of the said nucleotide sequence, or conversely, such that one of these primers is identical with the last 10 to 25 nucleotides of the said sequence whereas the other primer is complementary to the first 10 to 25 nucleotides (or hybridizes with the first 10 to 25 nucleotides) of the said nucleotide sequence, followed by the introduction of the said nucleotide sequences thus amplified into a suitable vector,
- the placing in culture in a suitable culture medium of a cell host previously transformed by a suitable vector containing a nucleic acid according to Claim 11 or 12, and
- the recovery from the above-mentioned culture medium of the polypeptide produced by said transformed cell host.

18. Nucleotide detection probe characterized in that it is constituted by all or part of a nucleotide sequence according to one of the Claims 5 to 7.

19. Method of in vitro diagnosis of malaria in an individual likely to be infected by P. falciparum which comprises the following steps :
- optional prior amplification of the quantity of nucleotide sequences according to any one of the Claims 5 to 7 likely to be contained in the biological sample taken from said individual with the aid of a DNA primer according to Claim 8 and a DNA primer according to Claim 9 or 10, and selected as indicated in Claim 17,
- the placing of the biological sample mentioned above in contact with a nucleotide probe according to Claim 18 under conditions permitting the production of a hybridization complex formed between the said probe and the said nucleotide sequence,
- the detection of the above-mentioned hybridization complex possibly formed.

20. Method of in vitro diagnosis of malaria in an individual likely to be infected by P. falciparum which comprises the placing of a tissue or a biological fluid taken from an individual in contact with a polypeptide according to one of the Claims 1 to 4 under conditions making possible an in vitro immunological reaction between the said polypeptide and the antibodies possibly present in the biological tissue and the in vitro detection of the antigen-antibody complex possibly formed.

21. Necessary or kit for the implementation of the in vitro diagnostic method according to Claim 19, characterized in that it contains :
- a defined quantity of a nucleotide probe according to Claim 18,
- advantageously a medium suitable for the formation of a hybridization reaction between the sequence to be detected and the above-mentioned probe,
- advantageously reagents making possible the detection of the hybridization complexes formed between the nucleotide sequence and the probe during the hybridization reaction.

22. Necessary or kit for the in vitro diagnosis of malaria in an individual likely to be infected by P. falciparum according to Claim 20 which comprises :
- a polypeptide according to one of the Claims 1 to 4,
- the reagents for the constitution of the medium suitable for carrying out the immunological reaction,
- the reagents making possible the detection of the antigen-antibody complex produced by the immunological reaction, such reagents being also capable of being labelled or being capable of being recognized in turn by a labelled reagent, more particularly in the case in which the above-mentioned polypeptide is not labelled.

23. The monoclonal or polyclonal antibodies which specifically recognize the peptide sequence according to one of the Claims 1 to 4.

24. Immunogenic composition characterized by a polypeptide in conformity with one of the Claims 1 to 4 in combination with a pharmaceutically acceptable vehicle.

25. Vaccinating composition directed against malaria, containing among other immunogenic principles a polypeptide in conformity with one of the Claims 1 to 4.

## Patentansprüche

1. Polypeptid, erzeugt durch den Bakterien-Stamm, transformiert durch das Plasmid DG-671 und hinterlegt am 31.03.1989 beim CNCM unter der Nummer I-855.

2. Polypeptid gemäß Anspruch 1,
dadurch **gekennzeichnet**, daß
es aus einem monomeren Peptid zusammengesetzt ist.

3. Polypeptid gemäß Anspruch 1,
dargestellt durch die folgende vollständige oder teilweise Verkettung von Aminosäuren:

Asn-Gly-Lys-Asp-Asp-Val-Lys-Glu-Glu-Lys-Lys-Thr-Asn-
Glu-Lys-Lys-Asp-Asp-Gly-Lys-Thr-Asp-Lys-Val-Gln-Glu-
Lys-Val-Leu-Glu-Lys-Ser-Pro-Lys-Glu-Phe.

4. Polypeptid,
dadurch **gekennzeichnet**, daß
es durch die im Plasmid DG-671 enthaltene Nukleinsäure kodiert ist, welches zur Transformierung des Bakterien-Stamms verwendet wurde, der am 31.03.1989 beim CNCM unter der Nr. I-855 hinterlegt wurde.

5. Nukleotidsequenz,
dadurch **gekennzeichnet**, daß
sie eine Verkettung von Nukleotiden enthält, die Polypeptide gemäß einem der Ansprüche 1 bis 4 kodieren.

6. Sequenz von Nukleotiden,
enthalten im Plasmid DG-671, verwendet zur Transformierung des Bakterien-Stamms, der am 31.03.1989 beim CNCM unter der Nr. I-855 hinterlegt wurde.

7. Sequenz von Nukleotiden, die dazu befähigt ist, mit der Nukleotidsequenz des Anspruchs 6 hybridisiert zu werden, oder die Sequenz, die zur letzteren unter den folgenden Bedingungen komplementär ist:
- Vorbehandlung (Vorhybridisierung) des Nitrozellulose-Filters, der das Nukleinsäure-Fragment trägt, das mit dem Hybridisierungs-Puffer (zusammengesetzt aus 6 x SSC, 2,5% Milch, 0,5% SDS) zu testen ist, wobei diese Verfahrensmaßnahme bei 65°C 1 h lang durchgeführt wird;
- Ersatz des Hybridisierungs-Puffers, der sich in Kontakt mit der Trägerunterlage befindet, an welche das Nukleinsäure-Fragment schließlich fixiert ist, durch Hybridisierungs-Puffer derselben Zusammensetzung (enthaltend außerdem DNA von denaturiertem Lachs) und Zugabe der Sequenz des Anspruchs 6 als Sonde, die insbesondere radioaktiv markiert und vorab durch eine Behandlung bei 100°C 5 Minuten lang denaturiert ist;
- Inkubation des genannten am Träger fixierten Nukleinsäure-Fragments mit der Sequenz der Figur 1 in diesem Inkubations-Puffer bei 65°C während einer Dauer von 16 bis 24 Stunden;
- Beseitigung des nicht-fixierte Sonde enthaltenden Puffers durch drei aufeinanderfolgende Wäschen von 5 Minuten mit jeweils 2 x SSC, 0,1% SDS bei 65°C und im Anschluß daran durch drei aufeinanderfolgende Wäschen von 5 Minuten mit jeweils 0,1 x SSC, 0,1% SDS bei 65°C.

8. Teilstück einer DNA oder RNA,
dadurch **gekennzeichnet**, daß
es aus ungefähr 10 bis 25 Nukleotiden zusammengesetzt ist, die mit den 10 bis 25 ersten Nukleotiden der Nukleotid-Sequenz, die eine Peptid-Sequenz gemäß einem der Ansprüche 1 bis 4 kodieren, oder mit den 10 bis 25 letzten Nukleotiden der genannten Sequenz identisch sind.

9. Teilstück einer DNA oder RNA,
dadurch **gekennzeichnet**, daß
es aus ungefähr 10 bis 25 Nukleotiden zusammengesetzt ist, die zu den 10 bis 25 ersten Nukleotiden der Nukleotid-Sequenz, die eine Peptid-Sequenz gemäß einem der Ansprüche 1 bis 4 kodieren, oder zu den 10 bis 25 letzten Nukleotiden der genannten Sequenz von Nukleotiden komplementär sind.

10. Teilstück einer DNA oder RNA gemäß Anspruch 9,
dadurch **gekennzeichnet**, daß
es aus ungefähr 10 bis 25 Nukleotiden zusammengesetzt ist, die dazu befähigt sind, mit den 10 bis 25 ersten Nukleotiden oder den 10 bis 25 letzten Nukleotiden der genannten Sequenz von Nukleotiden, die eine Peptid-Sequenz gemäß einem der Ansprüche 1 bis 4 kodieren, bei den in Anspruch 7 definierten Hybridisierungs-Bedingungen hybridisiert zu werden.

11. Rekombinante Nukleinsäure,
dadurch **gekennzeichnet**, daß
sie mindestens eine Sequenz von Nukleotiden gemäß einem der Ansprüche 5 bis 7 enthält, die in eine gegenüber der oben genannten Sequenz heterologe Nukleinsäure inseriert ist.

12. Rekombinante Nukleinsäure gemäß Anspruch 11,
dadurch **gekennzeichnet**, daß
der Sequenz von Nukleotiden gemäß einem der Ansprüche 5 bis 7 ein Promotor vorausgeschaltet ist, unter dessen Steuerung sich die Transkription der genannten Sequenz durchführen läßt, und daß, gegebenenfalls, eine Sequenz folgt, die Signale einer Beendigung der Transkription kodiert.

13. Rekombinanter Vektor, insbesondere zum Klonieren und/oder Exprimieren, nämlich vom Plasmid-, Cosmid- oder Phagen-Typ,
dadurch **gekennzeichnet**, daß
er eine rekombinante Nukleinsäure gemäß Anspruch 11 oder 12 an einer seiner Stellen enthält, die für seine Replikation nicht wesentlich sind.

14. Rekombinanter Vektor gemaß Anspruch 13,
dadurch **gekennzeichnet**, daß
es sich um das Plasmid DG-671 handelt, das am 31.03.1989 beim CNCM unter der Nummer I-855 hinterlegt wurde.

15. Rekombinanter Vektor gemäß Anspruch 13,
dadurch **gekennzeichnet**, daß
er an einer seiner, für seine Replikation nicht wesentlichen Stellen notwendige Elemente zum Promovieren der Expression einer peptidischen Sequenz gemäß einem der Ansprüche 1 bis 4 in einem zellulären Wirt und gegebenenfalls einen Promotor, der durch die Polymerasen des zellulären Wirts erkannt wird, insbesondere einen induktiblen Promotor, enthält.

16. Zellulärer Wirt, der durch einen rekombinanten Vektor gemäß einem jeden der Ansprüche 13 bis 15 transformiert wird und die Regelungselemente aufweist, die die Expression der Sequenz von Nukleotiden ermöglichen, die ein Polypeptid gemäß einem der Ansprüche 1 bis 4 in dieser Wirtszelle kodiert.

17. Verfahren zur Herstellung eines Polypeptids gemäß einem der Ansprüche 1 bis 4, umfassend die folgenden Stufen:
- gegebenenfalls, vorgeschaltete Vervielfältigung gemäß der PCR-Technik der Menge von Nukleotid-Sequenzen, die das genannte Polypeptid kodieren, mittels eines Teilstücks einer DNA gemäß Anspruch 8 oder eines Teilstücks einer DNA gemäß Anspruch 9 oder 10, welche so ausgewählt sind, daß das eine dieser Teilstücke mit den 10 bis 25 ersten Nukleotiden der Nukleotid-Sequenz, die das genannte Polypeptid kodieren, identisch ist, während das andere Teilstück zu den 10 bis 25 letzten Nukleotiden der genannten Nukleotid-Sequenz komplementär ist (oder mit diesen 10 bis 25 letzten Nukleotiden hybridisiert wird), oder welche umgekehrt so ausgewählt sind, daß das eine dieser Teilstücke mit den 10 bis 25 letzten Nukleotiden der genannten Sequenz identisch ist, während das andere Teilstück zu den 10 bis 25 ersten Nukleotiden der genannten Nukleotid-Sequenz komplementär ist (oder mit den 10 bis 25 ersten Nukleotiden hybridisiert wird), worauf die Einführung der genannten, auf diese Weise vermehrten Sequenzen von Nukleotiden in einen geeigneten Vektor erfolgt,
- Züchtung, in einem geeigneten Kulturmedium, einer Wirtszelle, die vorab durch einen geeigneten Vektor transformiert ist, der eine Nukleinsäure gemäß Anspruch 11 oder 12 enthält, und
- Gewinnung des aus der genannten transformierten Wirtszelle erzeugten Polypeptids aus dem oben genannten Kulturmedium.

18. Nukleotidische Nachweis-Sonde,
dadurch **gekennzeichnet**, daß
sie gänzlich oder teilweise aus einer Sequenz von Nukleotiden gemäß einem der Ansprüche 5 bis 7 zusammengesetzt ist.

19. In vitro-Diagnoseverfahren von Malaria bei einem Individuum, das dafür empfänglich ist, von P. falciparum infiziert zu werden, wobei das Verfahren die folgenden Stufen umfaßt:
- gegebenenfalls vorgeschaltete Vermehrung der Menge von Sequenzen von Nukleotiden gemäß einem jeden der Ansprüche 5 bis 7, die sich dazu eignen, in einem beim genannten Individuum vorgenommenen biologischen Assayverfahren enthalten zu sein, mittels eines Telstücks der DNA gemäß Anspruch 8 und eines Teilstücks der DNA gemäß Anspruch 9 oder 10, welche in der in Ansprüch 17 angegebenen Weise ausgewählt sind,
- Zusammenbringen der oben genannten biologischen Assay-Probe mit einer nukleotidischen Sonde gemäß Anspruch 18 unter Bedingungen, die die Erzeugung eines Hybridisierungs-Komplexes ermöglichen, der sich zwischen der genannten Sonde und der genannten Sequenz von Nukleotiden bildet,
- Nachweis des oben genannten Hybridisierungs-Komplexes der sich in entsprechender Weise gebildet hat.

20. In vitro-Diagnoseverfahren von Malaria bei einem Individuum, das dafür empfänglich ist, von P. falciparum infiziert zu werden, wobei man ein Gewebe oder eine biologische Flüssigkeit, welche von einem Individuum entnommen sind, mit einem Polypeptid gemäß einem der Ansprüche 1 bis 4 unter Bedingungen in Kontakt bringt, die eine immunologische in vitro-Reaktion zwischen dem genannten Polypeptid und den im biologischen Gewebe gegebenenfalls und/oder in entsprechender Weise vorhandenen Antikörpern ermöglichen, und wobei man den Antigen-Antikörper-Komplex, der gegebenenfalls und/oder in entsprechender Weise gebildet ist, in vitro nachweist.

21. Necessaire oder Kit zur Durchführung des in vitro-Diagnoseverfahrens gemäß Anspruch 19,
dadurch **gekennzeichnet**, daß
sie umfassen:
- eine bestimmte Menge einer nukleotidischen Sonde gemäß Anspruch 18;
- ein in vorteilhafter Weise vorliegendes Milieu, das sich zur Ausgestaltung einer Hybridisierungsreaktion zwischen der nachzuweisenden Sequenz und der oben genannten Sonde eignet,
- in vorteilhafter Weise vorliegende Reaktionsmittel, die den Nachweis von Hybridisierungs-Komplexen ermöglichen, die zwischen der Sequenz von Nukleotiden und der Sonde bei der Hybridisierungsreaktion gebildet sind.

22. Necessaire oder Kit für die in vitro-Diagnostik gemäß Anspruch 20 der Malaria bei einem Individuum, das dafür empfänglich ist, von P. falciparum infiziert zu werden, welche umfassen:
- ein Polypeptid gemäß einem der Ansprüche 1 bis 4,
- Reaktionsmittel zur Erstellung eines Milieu, das die Durchführung der immunologischen Reaktion begünstigt,
- Reaktionsmittel zum Nachweis des Antigen-Antikörper-Komplexes, der durch die immunologische Reaktion erzeugt ist, wobei diese Reaktionsmittel auch ein Markierungsmittel aufweisen können, oder wobei sie sich dazu eignen, ihrerseits durch ein markiertes Reaktionsmittel erkannt zu werden, insbesondere in dem Fall, bei dem das oben genannte Polypeptid nicht markiert ist.

23. Polyklonale oder monoklonale Antikörper, welche die peptidische Sequenz gemäß einem der Ansprüche 1 bis 4 spezifisch erkennen.

24. Immunogene Zusammensetzung,
**gekennzeichnet** durch
eine Zubereitungsform des Polypeptids gemäß einem der Ansprüche 1 bis 4, zusammen mit einem pharmazeutisch zulässigen Trägermittel.

25. Impfstoff-Zusammensetzung gegen Malaria, enthaltend neben weiteren immunogenen Wirkprinzipien das Polypeptid gemäß einem der Ansprüche 1 bis 4.
